# EUROPÄISCHE PATENTANMELDUNG

(11) **EP 0 922 459 A1**
(43) Veröffentlichungstag der Anmeldung: **16.06.1999**
(21) Anmeldenummer: 98123447.9
(22) Anmeldetag: 11.12.1998
(51) Int. Cl.: A61K 31/70, A61K 31/195, A61K 31/19

(54) **Pharmazeutische Zusammensetzung enthaltend D-Galaktose und ihre Verwendung**

(30) Priorität: 12.12.1997 DE 19755367
(71) Anmelder: Afting, Ernst-Günter, Prof. Dr. Dr., 82152 Krailling (DE); Reutter, Werner, Prof. Dr., D-14195 Berlin (DE)
(72) Erfinder: Afting, Ernst-Günter, Prof. Dr. Dr., 82152 Krailling (DE); Reutter, Werner, Prof. Dr., D-14195 Berlin (DE)
(74) Vertreter: Bösl, Raphael, Dr. rer. nat., Dipl.-Chem.

(57) **Zusammenfassung**

Die Erfindung betrifft pharmazeutische Zusammensetzungen enthaltend D-Galaktose und gegebenenfalls α-Ketoglutarsäure oder ein Salz davon sowie Ornithin oder ein Salz davon in Form von enteralen bzw. parenteralen Formulierungen zur Behandlung von Patienten in metabolischen Streßsituationen.

## Beschreibung

Die vorliegende Erfindung betrifft pharmazeutische Zusammensetzungen enthaltend D-Galaktose und gegebenenfalls α-Ketoglutarsäure oder ein Salz davon sowie Ornithin oder ein Salz davon in Form von enteralen bzw. parenteralen Formulierungen zur Behandlung von Patienten in metabolischen Streßsituationen.

Für die Aufrechterhaltung lebenswichtiger Funktionen einer Zelle ist es ein primäres Ziel einer jeden Zelle insbesondere in einer metabolischen Streßsituation, genügend Energie für die Aufrechterhaltung lebenswichtiger Funktionen zur Verfügung zu stellen.

Glukose ist als Energiespender besonders geeignet, da Glukose in der Glykolyse entsprechende Energieäquivalente in Form von NADH liefen. In der sogenannten Atmungskette wird mit Hilfe von NADH ATP gebildet, das als primärer Energieträger für die Funktion einer Zelle notwendig ist. Ein weiteres Produkt der Glykolyse ist Pyruvat, aus dem mitochondrial Acetyl-CoA gebildet wird, das in den sogenannten Citratcyclus eingeht und weitere NADH-Moleküle bildet. Wegen des großen Bedarfs an ATP muß die Zelle rasch und möglichst quantitativ Glukose in Pyruvat und Acetyl-CoA für den Citratcyclus umwandeln. Da pro Mol Acetyl-CoA 3 Mol NADH und anschließend in der Atmungskette aus 1 Mol NADH 3 Mol ATP gebildet werden, ergibt sich eine Energiebilanz von 36 Mol ATP pro Mol Glukose bzw. 82 g ATP aus 1 g Glukose.

Bei metabolischen Streßsituationen reicht in der Regel der Glukosegehalt der Nahrung zum Energiestoffwechsel und zur Erhaltung des Baustoffwechsels nicht aus. Insbesondere Alkoholiker leben wegen ihrer unausgewogenen Ernährung in einer latenten energetischen Unterversorgung (Hungerzustand). Durch diesen Hungerzustand verarmen zusätzlich essentiell wichtige "Seitenwege" des Glukose-Abbaus (Glykolyse). Diese Seitenwege dienen fast ausschließlich dem Baustoftwechsel der Zelle, d. h. der Bildung von integralen Zellbestandteilen wie intrazellulären Membranen und die Zelle umhüllende Plasmamembranen. Das betrifft auch die Umwandlung von Fructose-6-phosphat, einem weiteren Metaboliten der Glykolyse, zu Mannose-6-phosphat, die Umwandlung zu Aminozuckern, wie z. B. Glucosamin-6-phosphat und nachfolgenden Verbindungen bis zum N-Acetylglucosamin oder zur N-Acetylneuraminsäure, und die Umwandlung von aktivierter Glukose in Form von UDP-Glukose zur aktivierten Galaktose in Form von UDP-Galaktose.

Aufgrund metabolischer Streßsituationen gelangen somit wesentliche Teile des Baustoffwechsels in einen Mangelzustand, wobei besonders Glykoproteine und Glykolipide, z. B. Ganglioside, in den genannten Membranstrukturen betroffen sind. Hierdurch werden zudem eine Vielzahl von essentiellen zellulären Funktionen, die von Membranen ausgehen oder in Membranen lokalisiert sind, negativ beeinflußt. So gehört hierzu beispielsweise die Funktionsweise von Rezeptoren einer Zelle, z. B. von Hormonrezeptoren, Wachstumsfaktorrezeptoren und von Rezeptoren für den Zell-Zell-Kontakt, ohne die eine Zelle auf Dauer nicht überleben kann. Weitere essentielle zelluläre Funktionen sind in membrangebundenen Proteinen, Enzymen und Transportsystemen verkörpert, die auch die Antigenität einer Zelle repräsentieren. Zusammengefaßt regulieren die Rezeptoren die Informationsaufnahme und -weitergabe einer Zelle und geben ihr darüberhinaus das jeweils charakteristische, biochemische und strukturelle Aussehen.

Man hat daher versucht, durch enterale Gabe von Glukose- und Aminosäuregemischen den notwendigen Kalorienbedarf (Energiestoffwechsel) bei Patienten zu decken, die sich in einer ausgeprägten metabolischen Streß-Situation befinden.

Um die Versorgung des Baustoffwechsels bei Patienten mit metabolischem Defizit sicherzustellen, ist jedoch eine Versorgung zweckmäßig, die statt bzw. neben Glukose einen Baustein enthält, mit dem unmittelbar die Störung der essentiellen Funktionen von Membrankomponenten und von Rezeptoren oder Transportsystemen des Stoffwechsels rasch behoben werden kann.

DE 39 35 906 schlägt nun eine Präparation aus verschiedenen Glykosen und gegebenenfalls weiteren Bestandteilen vor, die für Infusionen und Injektionen zur parenteralen Ernährung und Versorgung von Patienten, die eine Intensivbehandlung benötigen bzw. unter metabolischem Streß leiden, geeignet ist, wobei mindestens 50 Mol-% der Glykosen von D-Galaktose gestellt werden.

Erst kürzlich wurde auch die parenterale Verwendung von Galaktose zur Verhinderung der Metastasenbildung von Kolonkarzinomen beschrieben, die auf eine Blockierung von Leberlektinen durch Galaktose zurückgeht (Warczynski, P. et al., 1997, Anticancer Res., 17, 1223-1226).

D-Galaktose ist bekanntlich ein Aldohexose-Isomeres der D-Glukose, das im Milchzucker in Form des Disaccharids Lactose und in einigen Gummiarten in Form von Galaktanen vorkommt. D-Galaktose wird ferner sehr rasch über D-Galaktose-1-phosphat in UDP-Galaktose umgewandelt.

Ein Nachteil der bekannten Präparationen mit Galaktose bzw. mit den Disacchariden Lactose oder Lactulose ist jedoch, daß sie nicht auf die hohen Spiegel von Ammoniumionen (NH₄⁺) bei Stoffwechselentgleisung, Leberversagen und anderen Streßsituationen sowie auf andere Giftstoffe abgestimmt sind. Zudem sind Disaccharide oder andere Vorläufermoleküle von Galaktose für die therapeutische Verwendung nicht geeignet, da hieraus Galaktose nur ohne die langsamere Disaccharid-Spaltung im Darmlumen verzögert freigesetzt wird. Ein therapeutisch wirksamer Galaktose-Spiegel im Körper kann hierdurch nicht erreicht werden. Für eine therapeutische Wirksamkeit ist es notwendig, einen effektiven Galaktose-Spiegel schnell zu erreichen (Bolus-Anflutung).

Aufgabe der vorliegenden Erfindung war daher, eine pharmazeutische Zusammensetzung zu finden, die metabolische Streß-Situationen bei Patienten behebt oder effektiver verringert und die beschriebenen Nachteile der bekannten Präparation vermeidet.

Es wurde nun überraschenderweise gefunden, daß sich der Allgemeinzustand komatöser Patienten durch insbesondere hohe Gaben von Galaktose und vor allem durch den Zusatz von α-Ketoglutarat und Ornithin zu Galaktosepräparaten schneller bessert und die Stress-Situation zum Wohle des Patienten effektiver abbaut als mit den bereits bekannten Präparationen. Der positive Effekt wird insbesondere dann festgestellt, wenn die Galaktosekonzentration im Blut schnell anflutet. Ein schnelles Anfluten der Galaktosekonzentration konnte beispielsweise dadurch erreicht werden, daß dem Patienten Galaktose entweder in fester Form parenteral oder als Bolus intravenös verabreicht wurde.

Ein Gegenstand der vorliegenden Erfindung ist daher eine pharmazeutische Zu-Sammensetzung in fester Form enthaltend D-Galaktose und gegebenenfalls α-Ketoglutarsäure oder ein Salz davon sowie Ornithin oder ein Salz davon.

Die Resorption der erfindungsgemäßen Zusammensetzung nach Gabe in fester Form erfolgt überraschend schnell, insbesondere wenn sie auf nüchternen Magen genommen wird. Das hat zur Folge, daß gängige Infusionslösungen mit Galaktose durch die kostengünstigere und den Patienten weniger belastende erfindungsgemäße Zusammensetzung ersetzt werden kann.

Als Salz eignet sich insbesondere das Natrium-, Kalium-, Magnesium-, Zink- und/oder Calciumsalz. Das Calciumsalz hat zusätzlich den Vorteil, daß gleichzeitig die Gefäße abgedichtet werden. Das Zinksalz hat den Vorteil, daß es ein potentielles Defizit der Zink-abhängigen Enzyme des Ammonium-entgiftenden Harnstoff-Cyclus ausgleicht.

α-Ketoglutarsäure und Ornithin sind als Zusatzstoffe besonders vorteilhaft im Sinne der vorliegenden Erfindung geeignet, da sie z. B. giftige Ammoniumionen abfangen, welche in untoxische Aminocarbonsäuren überführt und in der untoxischen Form im Harnstoff-Cyclus als Harnstoff gebunden werden, der dann ausgeschieden wird. Zudem können die Zusatzstoffe zusammen mit D-Galaktose schnell und problemlos enteral verabreicht werden, was besonders vorteilhaft ist, da die Konzentration der Wirkstoffe im Körper schnell anflutet und so in kurzer Zeit hochwirksame Spiegel von z. B. UDP-Galaktose erzeugt werden und der Harnstoff-Cyclus optimal mit Substraten zur Ammonium-Entgiftung versorgt wird.

In einer bevorzugten Ausführungsform liegt D-Galaktose beispielsweise in einer Menge von ca. 1-20 g, vorzugsweise ca. 2-18 g, insbesondere ca. 3-12 g, vor allem ca. 3-10 g pro Applikationsform vor. α-Ketoglutarsäure oder ein Salz davon liegt beispielsweise in einer Menge von ca. 50-300 mg, vorzugsweise ca. 75-250 mg, insbesondere ca. 100-200 mg, vor allem ca. 150 mg pro Applikationsform vor und Ornithin oder ein Salz davon in einer Menge von ca. 50-300 mg, vorzugsweise ca. 75-250 mg, insbesondere ca. 100-200 mg, vor allem ca. 150 mg pro Applikationsform.

Eine besonders bevorzugte Ausführungform ist eine erfindungsgemäße Zusammensetzung in Form einer Tablette, eines Pulvers oder eines Sirups, insbesondere in Form einer Brausetablette, da hierdurch eine Selbstmedikation besonders einfach möglich ist.

Ein anderer Gegenstand der vorliegenden Erfindung ist eine pharmazeutische Zusammensetzung in flüssiger Form enthaltend D-Galaktose, α-Ketoglutarsäure oder ein Salz davon sowie Ornithin oder ein Salz davon. Als Salz eignet sich wiederum insbesondere das Natrium-, Kalium-, Magnesium-, Zink- und/oder Calciumsalz.

In einer bevorzugten Ausführungsform liegt D-Galaktose beispielsweise in einer Konzentration von ca. 5-230 g D-Galaktos/250 ml, vorzugsweise in einer Konzentration von ca. 25-180 g D-Galaktose/250 ml, insbesondere in einer Konzentration von ca. 50-100 g D-Galaktose/250 ml, vor allem in einer Konzentration von ca. 70 g D-Galaktos/250 ml vor Zur Zubereitung der erfindungsgemäßen Zusammensetzung kann beispielsweise von einer 50%igen Galaktoselösung ausgegangen werden. α-Ketoglutarsäure oder ein Salz davon liegt beispielsweise in einer Konzentration von ca. 5-100 mM, vorzugsweise ca. 10-75 mM, insbesondere ca. 20-50 mM, vor allem ca. 25 mM vor und Ornithin oder ein Salz davon beispielsweise in einer Konzentration von ca. 1-20 mM, vorzugsweise ca. 2-15 mM, insbesondere ca. 3-10 mM, vor allem ca. 5 mM.

Eine besonders bevorzugte Ausführungsform ist es, wenn die erfindungsgemäße Zusammensetzung in Form einer Infusionslösung vorliegt, da diese entweder direkt oder über einen Bypass als Zusatz zu einer bereits existierenden Infusionslösung dem Patienten intravenös gegeben werden kann. Hierdurch können insbesondere größere Mengen der erfindungsgemäßen Zusammensetzung z. B. als Bolus verabreicht werden, um die vorteilhaften Wirkungen der Zusammensetzung besonders schnell erreichen zu können.

Desweiteren kann die erfindungsgemäße Zusammensetzung geeignete Zusätze und Hilfsstoffe, wie Monosaccharide, Vitamine, anorganische Elektrolyte, insbesondere ein Magnesium- und/oder Zinksalz in metabolisch notwendigen Dosen, Aromastoffe und Zusätze zur pH-Regulierung, Pufferung, Stabilisierung und Konservierung enthalten, wobei beispielsweise ca. 5-100 Gew.-%, vorzugsweise mindestens ca. 10-100 Gew-%, insbesondere ca. 20-100 Gew.-%, vor allem ca. 20 Gew-% des Monosaccharidanteils eine Verbindung ausgewählt aus D-Galaktose, D-Mannose, D-Glucosamin, D-Lactose und/oder D-Lactulose oder Gemische davon ist, wobei mindestens ca. 40 Mol-%, vorzugsweise mindestens ca. 50 Mol-%, insbesondere mindestens ca. 75 Mol-% dieser Verbindungen auf D-Galaktose entfallen soll. Im Falle der Disaccharide Lactose oder Lactulose werden diese wie Monosaccharide gerechnet. Dasselbe gilt für Invert- oder Rohrzuckerpräparate in bezug auf D-Glukose oder D-Fructose. Bei Vorhandensein von D-Galaktose und D-Mannose ist das Verhältnis von D-Galaktose zu D-Mannose in der erfindungsgemäßen Zusammensetzung im allgemeinen ca. 200:1 bis 2:1.

Soll die erfindungsgemäße Zusammensetzung in Form einer Brausetablette vorliegen, so eignet sich als weiterer Zusatzstoff z. B. Bicarbonat. Ein anderer Zusatzstoff, insbesondere bei einer Infusionslösung, kann γ-Globulin oder Albumin als Beispiel eines supplementierenden Proteins vorzugsweise in einer Menge von 0,05 bis 1 Gew.-% bezogen auf 100g Kohlenhydrate verwendet werden.

Als Vitamin eignet sich insbesondere Vitamin C, welches im Falle einer flüssigen Zusammensetzung in einer Konzentration von ca. 1-20 mM, vorzugsweise ca. 2-15 mM, insbesondere ca. 3-10 mM, vor allem ca. 5 mM vorliegt und im Falle einer festen Zusammensetzung in einer Menge von ca. 50-300 mg, vorzugsweise ca. 75-250 mg, insbesondere ca. 100-200 mg, vor allem ca. 150 mg.

Besonders bevorzugt ist es, wenn die pharmazeutische Zusammensetzung neben D-Galaktose, α-Ketoglutarsäure oder ein Salz davon, Ornithin oder ein Salz davon auch Vitamin C enthält. Durch die Zusätze α-Ketoglutarsäure und Ornithin werden die Giftstoffe im Körper besonders schnell abgefangen. Vitamin C wirkt im Sinne der vorliegenden Erfindung als Reduktionsäquivalent. Reduktionsäquivalente liegen insbesondere in metabolischen Streßsituationen in verminderter Form vor. Insbesondere eignet sich diese erfindungsgemäße Zusammensetzung in Form einer Tablette, eines Pulvers oder Sirups, insbesondere in Form einer Brausetablette, da die Wirkstoffe besonders schnell im Körper anfluten und in das Gewebe penetrieren können, besonders wenn die erfindungsgemäße Zusammensetzung auf nüchternen Magen genommen wird.

Desweiteren kann die erfindungsgemäße Zusammensetzung neben Ornithin auch noch andere essentielle Aminosäuren beispielsweise in einer Konzentration von ca. 1-15 g/l vorzugsweise ca. 2-9 g/l je Aminosäure enthalten. Üblicherweise sind die zusätzlichen Aminosäuren und Ornithin in derselben Menge bzw. Konzentration vorhanden.

Als Elektrolyte sind beispielsweise Natriumchlorid, Kaliumchlorid, Calciumchlorid, Zinkchlorid und/oder Magnesiumchlorid geeignet, wobei das Verhältnis der einwertigen zu den zweiwertigen Kationen im allgemeinen im Bereich von ca. 35:1 bis 10:1 liegt. Die Lösung enthält im allgemeinen insgesamt bis zu ca. 200 mval/l Elektrolyte.

Untersuchungen an Ratten haben ferner überraschenderweise gezeigt, daß im Gehirn D-Galaktose innerhalb von wenigen Stunden zu Aminosäuren metabolisiert, besonders zu Glutaminsäure, Glutamin, γ-Aminobuttersäure (GABA) und Asparaginsäure, wobei hierfür Stickstoffäquivalente notwendig sind, die in vermehrten Umfang im Blut und damit auch im Gehirn bei Schädigung der Leber, z. B. bei Alkoholschädigung oder anderen chronischen, meist viral bedingten Entzündungen oder toxischen Leberschäden auftreten und abgefangen oder neutralisiert werden müssen.

Nach prä- und postoperativen Galaktosegaben (0,5 bis 1,0 g/kg Körpergewicht) an Menschen gemaß der vorliegenden Erfindung wurde im Blut der Patienten überraschenderweise ein Anstieg der Konzentration von Arginin gemessen. Dieser Befünd ist für den Organismus vorteilhaft, da aus Arginin der Transmitter Stickstoffmonoxid (NO) gebildet wird, der für die Regulation des Blutdrucks, die Stimulation der Angiogenese und die Funktion des Hypophysenvorderlappens (Ausschüttung von Wachstumshormon) wichtig ist. Außerdem entstehen aus Arginin Glutaminsäure und Glutamin. Weiterhin steigen nach Galaktosegabe verzweigtkettige Aminosäuren an, die den Einstrom von Substraten in das Gehirn hemmen, aus denen falsche Neurotransmitter, besonders bei hepatischer Enzephalopathie und anderen Streßsituationen gebildet werden.

Auch die Konzentration anderer glucoplastischer Aminosäuren im Blut steigt an. Daher kann durch die Galaktosesupplementation der Zusatz von glucoplastischen Aminosäuren, besonders in den Infusionen, eingeschränkt oder ersetzt werden.
Die vorliegende Erfindung bezieht sich daher auch auf die Verwendung der erfindungsgemäßen Zusammensetzung zur Erhöhung des Aminosäurespiegels im Blut, insbesondere zur Erhöhung des Spiegels an verzweigt-kettigen Aminosäuren, glucoplastischen Aminosäuren und/oder Arginin, vorzugsweise die Erhöhung des Argininspiegels.

Die erfindungsgemäßen Zusammensetzungen eignen sich daher in besonders vorteilhafter Weise zur Behandlung von Lebererkrankungen und metabolischem Leberkoma, aber auch generell zur Behandlung von allen Formen des metabolischen Stresses beispielsweise bei Intensivstation-Patienten.

Die vorliegende Erfindung bezieht sich daher auch auf die Verwendung der erfindungsgemäßen Zusammensetzung zur Behandlung von Patienten mit metabolischen Streßsituationen, insbesondere zur Behandlung Lebererkrankungen, vor allem zur Behandlung von Patienten mit hepatischem Koma, nekrotisierender Pankreatitis, zur Prophylaxe und Therapie eines alkoholischen Leberschadens, zur Unterstützung der allgemeinen Leberfunktion, zur Unterstützung der Entgiftungsfunktion der Leber, zur Aufrechterhaltung der Leberfunktion und Leberstruktur, zur Unterstützung der Regeneration der geschädigten Leber sowie zur Verringerung der Nachwirkungen eines übermäßigen Alkoholkonsums ("Kater").

Ein weiteres Anwendungsgebiet der erfindungsgemäßen Zusammensetzung liegt bei Streßsituationen im Falle von hyperglykämischen Zuständen, z. B. bei Diabetes mellitus, vor. Interessanterweise gelingt es mit Hilfe der erfindungsgemäßen Zusammensetzung, die Hämoglobin-gebundenen Glukosewerte (HbA₁- und HbA_{1C}-Werte) relativ schnell wieder auf ein Normalniveau zu bringen.

Die vorliegende Erfindung bezieht sich daher auch auf die Verwendung der erfindungsgemäßen Zusammensetzung zur Normalisierung der Hämoglobingebundenen Glukosewerte bei Diabetes mellitus.

Ein weiteres Anwendungsgebiet der erfindungsgemäßen Zusammensetzung ist die Bekämpfung der Symptome der Alzheimer'schen Krankheit. Beim Morbus Alzheimer ist der Energiestoffwechsel im Gehirn allgemein vermindert. Folglich herrscht im Gehirn ein potentieller Substratmangel für den Energie- und Baustoffwechsel, insbesondere zur Synthese von Glykolipiden (Gangliosiden) und Glykoproteinen zur Wiederherstellung der Membran- und Nervenfunktionen.

Nach enteraler Gabe von 800 mg/kg Körpergewicht D-Galaktose wurde ein Anstieg des UDP-Galaktose-Spiegels in Körpergeweben, insbesondere im Gehirn, um einen Faktor von 2-3 gefunden. Der Spiegel an UDP-Glukose, die sich direkt aus UDP-Galaktose bilden kann, steigt ebenfalls an. Anschließend wird Galaktose, sofern sie nicht tur die Glykansynthese von Glykoproteinen und Glykolipiden verwendet wird, teilweise über die bekannten Stoffwechselwege, z. B. Citratcyclus, in Aminosäuren umgewandelt. Im Gehirn ist hier die Umwandlung in Glutaminsäure und GABA deutlich meßbar. Beide Substanzen sind essentiell für die Hirnfunktion, die bei Intensivstation-Patienten und Morbus Alzheimer-Patienten meist eingeschränkt ist. Zugleich wird dadurch erreicht, daß Aminogruppen über eine Aminierungsreaktion mit Galaktose bzw. gemäß der vorliegenden Erfindung in vorteilhafter Weise auch mit α-Ketoglutarat zu Glutamat/Glutamin verbraucht werden, was zu einer besonders effektiven Ammoniak-Entgiftung des Körpers führt, da Ammoniak Vorläufergruppen für die Bildung von Aminosäuren darstellt und via Glutamat/Glutamin über den Harnstoff-Cyclus entgiftet und ausgeschieden wird. Ammoniak fällt bei metabolischen Streßsituationen, vor allem bei Leberkoma in großen Mengen an und führt zur starken Beeinträchtigung zerebraler Funktionen (Enzephalopathien). Durch eine Bolusgabe von D-Galaktose, die zu einem schnellen Anfluten von Galaktose im Blutkreislauf und im Gehirn, d. h. zu einem schnellen Wirkspiegel, führt, wurde überraschenderweise eine rasche und merkliche Erholung der Patienten beobachtet. Diese Wirkung wird erfindungsgemäß in vorteilhafter Weise durch die gleichzeitige Gabe von α-Ketoglutarat und Ornithin verstärkt.

Daher bezieht sich die vorliegende Erfindung auch auf die Verwendung der erfindungsgemäßen Zusammensetzung zur Behandlung von Enzephalopathien.

Die Verwendung der erfindungsgemäßen Zusammensetzung ist auch deshalb so vorteilhaft, da sie praktisch frei von unerwünschten Nebenwirkungen und somit zur Selbstmedikation besonders geeignet ist. Überschüssige D-Galaktose wird sehr gut in Aminosäuren umgewandelt und hilft daher den Einsatz nicht-essentieller Aminosäuren z. B. auch in Infusionslösungen zu mindern. Überschüssige UDP-Galaktose als Metabolit von D-Galaktose wird durch Epimerisierung über UDP-Glukose in die Glykolyse und den nachfolgenden, bekannten Stoffwechselwegen eingeschleust und dient somit der Bereitstellung von Energie im Energiestoffwechsel, wie oben bereits näher erläutert.

Daher bezieht sich die vorliegende Erfindung auch auf die Verwendung der erfindungsgemäßen Zusammensetzung zur Behandlung von Eßstörungen, wie Kachexie, Bulimie oder Magersucht.

## Patentansprüche

1. Pharmazeutische Zusammensetzung in fester Form enthaltend D-Galaktose und vorzugsweise α-Ketoglutarsäure oder ein Salz davon sowie Ornithin oder ein Salz davon.

2. Pharmazeutische Zusammensetzung nach Anspruch 1, dadurch gekennzeichnet, daß D-Galaktose in einer Menge von ca. 1-20 g pro Applikationsform enthalten ist.

3. Pharmazeutische Zusammensetzung nach Anspruch 1 oder 2, dadurch gekennzeichnet, daß α-Ketoglutarsäure oder ein Salz davon in einer Menge von ca. 50-300 mg pro Applikationsform enthalten ist.

4. Pharmazeutische Zusammensetzung nach einem der Ansprüche 1-3, dadurch gekennzeichnet, daß Ornithin in einer Menge von ca. 50-300 mg pro Applikationsform enthalten ist.

5. Pharmazeutische Zusammensetzung nach einem der Ansprüche 1-4 in Form einer Tablette oder eines Pulvers, insbesondere in Form einer Brausetablette.

6. Pharmazeutische Zusammensetzung in flüssiger Form enthaltend D-Galaktose, α-Ketoglutarsäure oder ein Salz davon und Ornithin oder ein Salz davon.

7. Pharmazeutische Zusammensetzung nach Anspruch 6, dadurch gekennzeichnet, daß D-Galaktose in der Zusammensetzung in einer Konzentration von ca. 5-230 g/250 ml enthalten ist.

8. Pharmazeutische Zusammensetzung nach Anspruch 6 oder 7, dadurch gekennzeichnet, daß α-Ketoglutarsäure oder ein Salz davon in einer Konzentration von ca. 5-100 mM enthalten ist.

9. Pharmazeutische Zusammensetzung nach einem der Ansprüche 6-8, dadurch gekennzeichnet, daß Ornithin oder ein Salz davon in einer Konzentration von ca. 1-20 mM enthalten ist.

10. Pharmazeutische Zusammensetzung nach einem der Ansprüche 6-9 in Form einer Infusionslösung oder in Form eines Sirups.

11. Pharmazeutische Zusammensetzung nach einem der Ansprüche 1-10, dadurch gekennzeichnet, daß die Zusammensetzung zusätzlich Vitamin C enthält.

12. Pharmazeutische Zusammensetzung nach Anspruch 11, dadurch gekennzeichnet, daß Vitamin C im Falle einer flüssigen Zusammensetzung in einer Konzentration von ca. 1-20 mM vorliegt und im Falle einer festen Zusammensetzung in einer Menge von ca. 50-300 mg pro Applikationsform.

13. Pharmazeutische Zusammensetzung nach einem der Ansprüche 1-12, dadurch gekennzeichnet, daß das genannte Salz ein Natrium-, Kalium-, Magnesium-, Zink- und/oder Calciumsalz ist.

14. Pharmazeutische Zusammensetzung nach einem der Ansprüche 1-13, dadurch gekennzeichnet, daß die Zusammensetzung noch weitere geeignete Zusatz- und/oder Hilfsstoffe, insbesondere ein Magnesium- und/oder Zinksalz enthält.

15. Verwendung einer pharmazeutischen Zusammensetzung nach einem der Ansprüche 1-14 zur Erhöhung des Aminosäurespiegels im Blut, insbesondere zur Erhöhung des Spiegels an verzweigt-kettigen Aminosäuren, glucoplastischen Aminosäuren und/oder Arginin.

16. Verwendung einer pharmazeutischen Zusammensetzung nach einem der Ansprüche 1-14 zur Behandlung von Patienten in metabolischen Streßsituationen.

17. Verwendung einer pharmazeutischen Zusammensetzung nach einem der Ansprüche 1-14 zur Behandlung von Lebererkrankungen und metabolischem Leberkoma, zur Prophylaxe und Therapie eines alkoholischen Leberschadens, zur Unterstützung der allgemeinen Leberfunktion, zur Unterstützung der Entgiftungsfunktion der Leber, zur Aufrechterhaltung der Leberfunktion und Leberstruktur, zur Unterstützung der Regeneration der geschädigten Leber und/oder zur Verringerung der Nachwirkungen eines übermäßigen Alkoholkonsums.

18. Verwendung einer pharmazeutischen Zusammensetzung nach einem der Ansprüche 1-14 zur Normalisierung der Hämoglobin-gebundenen Glukosewerte.

19. Verwendung einer pharmazeutischen Zusammensetzung nach einem der Ansprüche 1-14 zur Behandlung von Enzephalopathien.

20. Verwendung einer pharmazeutischen Zusammensetzung nach einem der Ansprüche 1-14 zur Behandlung von Eßstörungen, insbesondere zur Behandlung von Kachexie, Bulimie oder Magersucht.
